# EUROPEAN PATENT APPLICATION

(11) **EP 2 713 293 A2**
(43) Date of publication of application: **02.04.2014**
(21) Application number: 13186465.4
(22) Date of filing: 27.09.2013
(51) Int. Cl.: G06F 19/00

(54) **Rapid community learning for predictive models of medical knowledge**

(30) Priority: 27.09.2012 US 201261706293 P; 18.10.2012 US 201261715447 P; 16.09.2013 US 201314027494
(71) Applicant: Siemens Medical Solutions USA, Inc., Malvern, PA 19355-1406 (US)
(72) Inventor: Anand, Vikram, Downingtown, PA 19335 (US); Farooq, Faisal, Norristown, PA 19403 (US); Krishnapuram, Balaji, King of Prussia, PA 19406 (US); Yu, Shipeng, Exton, PA 19341 (US)
(74) Representative: French, Clive Harry

(57) **Abstract**

A predictive model of medical knowledge is trained (42, 48) from patient data of multiple different medical centers (14). The predictive model is machine learnt from routine patient data from multiple medical centers (14). Distributed learning avoids transfer of the patient data from any of the medical centers (14). Each medical center (14) trains (42) the predictive model from the local patient data. The learned statistics, and not patient data, are transmitted (44) to a central server (12). The central server (12) reconciles (48) the statistics and proposes (52) new statistics to each of the local medical centers. In an iterative approach, the predictive model is developed without transfer of patient data but with statistics responsive to patient data available from multiple medical centers (14). To assure comfort with the process, the transmitted statistics may be in a human readable format.

## Description

### RELATED APPLICATIONS

The present patent document claims the benefit of the filing dates under 35 U.S.C. §119(e) ofProvisional U.S. Patent Application Serial No. 61/706,293, filed Sept. 27, 2012, and Provisional U.S. Patent Application Serial No. 61/715,447, filed Oct. 18, 2012, which are hereby incorporated by reference.

### FIELD

The present embodiments relate to rapid learning. A community of linked centers is used to make a medical predication useful for patient care.

### BACKGROUND

"Personalised treatment" is a buzz phrase, including in cancer treatment. While tailoring treatment to the individual patient has always been done to some extent, the promise of personalised approaches includes more effective therapies and improved treatment outcomes, and sparing patients the toxicity and cost associated with ineffective treatment.

The general assumption of personalised medicine is that one can split the patient population into ever smaller groups and that specific treatments have different outcomes between these groups. Successful cancer treatment requires an individual approach, in which diagnostic and treatment modalities are chosen according to the characteristics of an individual patient, his or her tumor and specific areas within the tumor. This individualized care does not sit well with the current, extremely costly method from basic research to clinical trial, which tries to identify if a novel modality is of benefit to a certain population of patients. As the treatments become more targeted and patients are more heavily selected, the controlled clinical trial approach to test these growing numbers of hypotheses and to support treatment decisions, becomes more difficult and costly.

Existing data may be used for in-silico trial testing of hypotheses about treatment, selection criteria for focusing controlled clinical trails or other predictions. Predictive modelling is more reliable with larger sample sets of routine or clinical patient data. For personalized medicine, the number of patients with similar circumstances at a given medical institution is limited. As the treatment becomes more personalized, data from fewer patients is available at a given medical institution.

The sharing of patient data between medical institutions is hampered by ethical, political and administrative barriers. Privacy concerns, the value (monetary, scientific, marketing) to institutions that hold the patient data, and the effort required to interpret, translate, annotate, and transfer the patient data from local databases are barriers for in-silico testing. Medical institutions are unlikely to be willing to export the patient data for aggregation to train better predictive models.

### SUMMARY

By way of introduction, the preferred embodiments described below include methods, instructions, and systems for learning a predictive model of medical knowledge. The predictive model is machine learnt from routine patient data from multiple medical centers. Distributed learning avoids transfer of the patient data from any of the medical centers. Each medical center trains the predictive model from the local patient data. The learned statistics, and not patient data, are transmitted to a central server. The central server reconciles the statistics and proposes new statistics to each of the local medical centers. In an iterative approach, the predictive model is developed without transfer of patient data but with statistics responsive to patient data available from multiple medical centers. To assure comfort with the process, the transmitted statistics may be in a human readable format.

In a first aspect, a method is provided for learning a predictive model of medical knowledge. First patient data in a first database of a first medical center is accessed. A first processor of the first medical center trains a first predictive model with the first patient data. The first parameters of the first predictive model are transmitted without transmitting the first patient data. The transmitting is to a server remote from first and second medical centers. Second patient data in a second database of a second medical center different than the first medical center is accessed. A second processor of the second medical center trains a second predictive model with the second patient data. Second parameters of the second predictive model are transmitted to the server without transmitting the second patient data. The server reconciles the first and second parameters into a third predictive model. Third parameters of the third predictive model are transmitted to the first and second medical centers. The first and second predictive models are re-trained at the first and second medical centers, respectively, as a function of the third parameters. Fourth and fifth parameters of the re-trained first and second predictive models are transmitted to the server. The server generates a fourth predictive model as a function of the fourth and fifth parameters.

In a second aspect, a non-transitory computer readable storage medium has stored therein data representing instructions executable by a programmed processor for learning a predictive model of medical knowledge. The storage medium includes instructions for receiving different sets of model values for the predictive model from different processors, the different sets of the model values from the different processors being machine learnt from clinical data for different sets of patients, the clinical data for the different sets of the patients not being received, generating consensus model values from the different sets of the model values without access to the clinical data, and transmitting the consensus model values to the different processors.

In a third aspect, a system is provided for learning a predictive model of medical knowledge. A central server is configured to communicate with a plurality of processors. The plurality of processors is for a respective plurality of different medical entities. Each of the processors is configured to generate local predictive models from medical data of the respective medical entity. The central server and processors are configured to perform distributed machine learning using the medical data from the different medical entities. The distributed machine learning results in a central predictive model learnt from the medical data of the plurality of the different medical entities while avoiding transfer of the medical data from any of the different medical entities.

The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.

Figure 1 is a block diagram of one embodiment of a system for learning a predictive model of medical knowledge;

Figure 2 is a flow chart diagram of one embodiment of a method for learning a predictive model of medical knowledge;

Figures 3 and 4 shows example messages transmitted from local medical centers to a central server; and

Figure 5 shows example messages transmitted from the central server to local medical centers.

### DETAILED DESCRIPTION

An information technology platform is provided for clinical cancer research or other predictive modeling from a community. The predictive model may be for determining a treatment or other care of a specific patient. The personalized characteristics of an individual patient and his or her condition (e.g., tumor) are taken into account in the care using the learned predictive model. Predictive models may be trained for determining selection criteria for clinical trials of new diagnostic and therapeutic modalities, for diagnosis, or for other medical predictions.

To train a predictive model from patient data of a community of different medical centers, there are various considerations. Local data extraction systems are developed and validated. The data extraction system extracts locally available medical data from all patients at each of the multiple centers. The medical data is mapped into a common terminology using a shared ontology. Effective and efficient information technology tools extract, browse, and query the relevant data from heterogeneous databases, and semantically normalize the data into a format that can be understood from other participating sites.

To obtain sources of patient data for as many patients as possible, a multi-centric infrastructure accesses the patient data locally. The predictive models are provided through a unified interface in a privacy-preserving manner where patient data does not leave the local institution as part of the learning. Distributed machine-learning avoids aggregating or transmission of patient specific data. Without copying data from existing databases and only linking them together via the privacy preserving mining infrastructure, learning on a larger scale is provided. Distributed learning from access to clinical data for a larger number of patients will improve the ability to learn and predict the outcome of individual treatments.

Machine learning-based predictive models for lung cancer or other conditions use various types of data (e.g., demographics, imaging, labs, genomics, etc) from multiple institutions while preserving privacy. Rather than predicting patient outcome for treatment, the machine learning-based predictive models may be used to simulate new treatments and identify useful selection criteria for a clinical trial and/or cost-effectiveness. An example of such an "in-silico trial" is a planning study, which compares various radiotherapy modalities (e.g., protons, carbon ions, photons 3D, photons IMRT, or tomotherapy) in terms of cost-efficiency. The predictive model may be used to find patients for trials and decrease and speed up the administration and analysis around clinical trials.

A shared database of various predictive models (e.g., medical characteristics in cancer patients, tumors and treatments) may be created. A data mining infrastructure for clinical trials, research, comparative effectiveness, or other purpose is developed and validated. The data mining infrastructure attracts medical companies, academic medical centers, hospitals, research organizations, or other entities to perform clinical research and development.

The discussion herein uses a cancer example. For example, the likelihood of survival after two years of a treatment is to be predicted. Given one or more characteristics of a patient, the predictive model indicates the chances of two-year survival using a given a specific cancer treatment. Models for predicting a best treatment, models for predicting determinative inclusion or exclusion criteria for a clinical trial, or other predictive models may be used for cancer related prediction. The predictive model may be trained to make medical related predictions for any condition, such as diseases other than cancer.

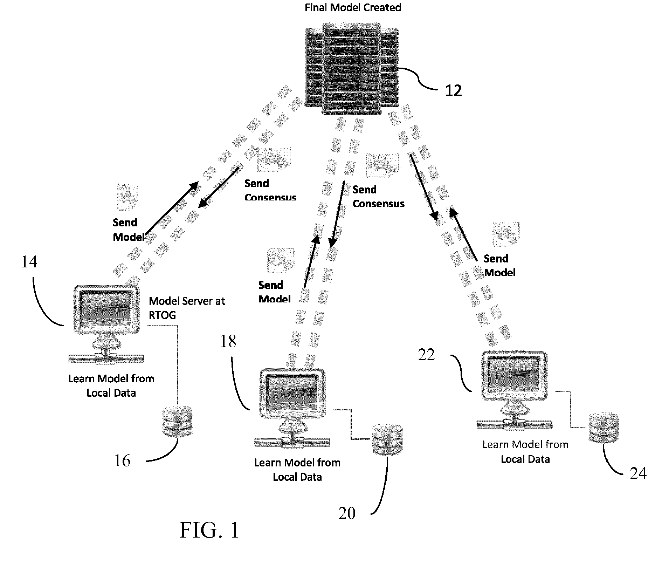

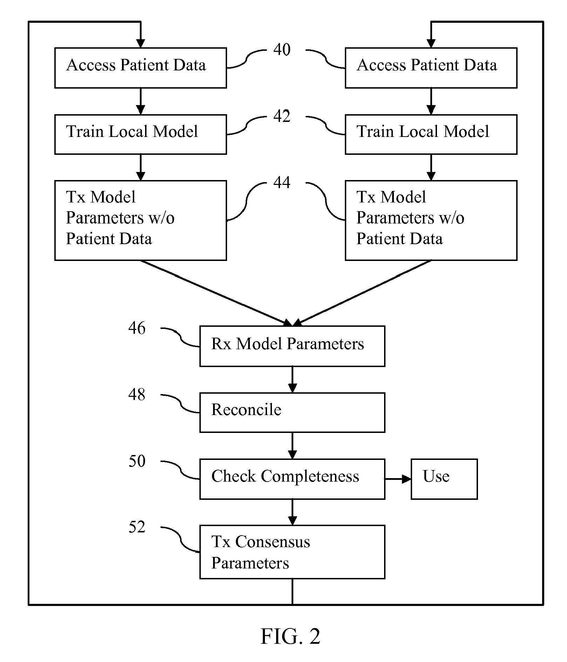

Figure 1 shows one embodiment of a system for learning a predictive model of medical knowledge. The system implements the method of Figure 2 or other methods. The system includes a central server 12 and a plurality of medical centers represented by the local servers 14, 18, and 22 and corresponding databases 16, 20, 24 of patient data. Additional, different or fewer components may be provided. For example, three medical centers are shown, but only two, four, or more medical centers may be used.

Each medical center is a hospital, institution, research facility, office, medical learning hospital, university, or other entity involved in storing patient medical data. The medical center may be involved in the treatment and/or diagnosis of patients. Routine data gathered for one or more patients is stored at each medical center. The storage may be off-site, but is "at" the medical center by being available for access at the medical center. Access outside the medical center is prevented or limited. For example, a hospital or organization of hospitals store patient data for patients being treated. Access to the patient data is restricted so that a different or unaffiliated doctor or hospital may not acquire the information without permissions.

The different medical centers have patient data for different sets of patients. The different medical centers may have the same or different standards of care, processes, treatments, patient approaches, or other care related approaches. Similarly, the types of patients (e.g., socio-economic, racial, or other differences) most common for the different medical centers may be similar or different. In one embodiment, the different medical centers are associated with treatment of patients in different counties, states, and/or countries.

The medical centers have one or more processors 14, 18, 22 and corresponding databases 16, 20, 24. In the example of Figure 1, one medical center is represented by one processor 14 and one database 16, another by processor 18 and database 20, and another by processor 22 and database 24. The processors 14, 18, 22 are local to (e.g., within a same building, campus, or facility) or remote from the databases 16, 20, 24. The processors 14, 18, 22 represent a given computer or server, but may be part of a network of computers or servers. Similarly, the databases 16, 20, 24 represent a given memory stack, but may be part of a network of databases. While one processor and one database is shown for each medical center, more than one processor and/or database may be involved in locally training a predictive model for a given medical center. The processor and database are representative.

The central server 12 is or is not affiliated or part of any of the medical centers. In one embodiment, the central server 12 is managed by a different entity than the medical centers and is a service provider of predictive models. The central server 12 is located in a different building, campus, region, or geographic location than any of the medical centers. In other embodiments, one or more of the medical centers create and manage the central server 12. The central server 12 may or may not share a campus, building, or facility with one of the medical centers.

The central server 12 and processors 14, 18, 22 are hardware devices with processing implemented in various forms of hardware, software, firmware, special purpose processors, or a combination thereof. Some embodiments are implemented in software as a program tangibly embodied on a program storage device. The central server 12 and processors 14, 18, 22 may each be a computer, personal computer, server, PACs workstation, imaging system, medical system, network processor, network, or other now know or later developed processing system. The central server 12 and processors 14, 18, 22 may each include at least one processor operatively coupled to other components. The processor is implemented on a computer platform having hardware components. The other components include a memory, a network interface, an external storage, an input/output interface, a display, and/or a user input. Additional, different, or fewer components may be provided. The computer platform may also include an operating system and microinstruction code. The various processes, methods, acts, and functions described herein may be part of the microinstruction code or part of a program (or combination thereof) which is executed via the operating system.

A user interface is provided for predictive modeling. The user interface is at the central server 12 and/or the processors 14, 18, 22. The user interface may be limited to configuring a predictive model and arranging for learning of the predictive model. In this configuration, access to patient data of particular patients is prevented. Instead, the user may select a type of predictive model, type of prediction, features for the predictive model, syntax to use for the predictive model, medical centers to participate, a collection or files storing patient data to be analyzed, or other information by selection, input, or from a menu. For application of the predictive model, the user interface may allow for access to patient data.

The user input may be a mouse, keyboard, track ball, touch screen, joystick, touch pad, buttons, knobs, sliders, combinations thereof, or other now known or later developed input device. The user input operates as part of a user interface. For example, one or more buttons are displayed on the display. The user input is used to control a pointer for selection and activation of the functions associated with the buttons. Alternatively, hard coded or fixed buttons may be used.

The user interface may include a display. The display is a CRT, LCD, plasma, projector, monitor, printer, or other output device for showing data.

The central server 12 and/or the processors 14, 18, 22 operate pursuant to instructions. The instructions and/or patient records for training a probabilistic prediction model are stored in a non-transitory computer readable memory such as an external storage, ROM, and/or RAM. The instructions for implementing the processes, methods and/or techniques discussed herein are provided on computer-readable storage media or memories, such as a cache, buffer, RAM, removable media, hard drive or other computer readable storage media. Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination. In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU or system. Because some of the constituent system components and method acts depicted in the accompanying figures may be implemented in software, the actual connections between the system components (or the process steps) may differ depending upon the manner of programming.

The same or different computer readable media may be used for the instructions, the patient data, and the predictive model. The patient records are stored in an external storage (databases 16, 20, 24), but may be in other memories. The external storage may be implemented using a database management system (DBMS) managed by the processor and residing on a memory, such as a hard disk, RAM, or removable media. Alternatively, the storage is internal to the processor (e.g. cache). The external storage may be implemented on one or more additional computer systems. For example, the external storage may include a data warehouse system residing on a separate computer system, a PACS system, or any other now known or later developed hospital, medical institution, medical office, testing facility, pharmacy or other medical patient record storage system. The external storage, an internal storage, other computer readable media, or combinations thereof store data for at least one patient record for a patient. The patient record data may be distributed among multiple storage devices.

The processors 14, 18, 22 and central server 12 has any suitable architecture, such as a general processor, central processing unit, digital signal processor, application specific integrated circuit, field programmable gate array, digital circuit, analog circuit, combinations thereof, or any other now known or later developed device for processing data. Likewise, processing strategies may include multiprocessing, multitasking, parallel processing, and the like. A program may be uploaded to, and executed by, the processor. The processor implements the program alone or includes multiple processors in a network or system for parallel or sequential processing.

In the arrangement of Figure 1, the central server 12 and/or the processors 14, 18, 22 communicate through one or more networks. Wired and/or wireless communications are used. The networks may be local area, wide area, public, private, enterprise, or other networks. Any communication format may be used, such as e-mail, text, or TCP/IP. Direct or indirection communication is provided. The communications may or may not be secured, such as using a public key infrastructure.

The processors 14, 18, 22 and central server 12 may perform the workflows, machine learning, model training, model application, and/or other processes described herein. For example, the processors 14, 18, 22 are configured to extract patient data and semantically normalize the medical data at the respective medical entities prior to performing the distributed machine learning. Each of the processors 14, 18, 22 is configured to generate a local predictive model from medical data available to the respective medical entity. The accessed patient data is used to generate model statistics representing the local predictive model. Due to the number of patients associated with the medical center, the local predictive model may or may not have sufficient training data to be reliable. The model statistics, rather than the patient data, is communicated to the central server 12.

The processors 14, 18, 22 may also be configured to apply trained probabilistic models, such as the local probabilistic model and a consensus probabilistic model. For applying the model, the model may have been trained by a different processor or the same processor. Feature values are extracted from patient data for a patient to be treated. The extracted feature values are input to the predictive model, which provides a prediction.

The central server 12 is configured to reconcile the learning of the probabilistic predictive models across the multiple medical centers. The central server 12 generates the central predictive model from the model statistics of the local or medical center predictive models. In an iterative process, the central server 12 may communicate consensus model statistics to the local medical centers for validation and further refinement based on the locally available patient data by the processors of the medical centers. The process repeats until convergence of the consensus model or another stop criterion is met.

The use of the central server 12 for reconciling and the local medical centers for training based on local patient data provides distributed machine learning using the medical data from the different medical entities. The distributed machine learning results in a central predictive model learnt from the medical data of the plurality of the different medical entities while avoiding transfer of the medical data from any of the different medical entities. The final predictive model is trained from patient data of multiple medical centers without any of the medical centers sharing the data with the other medical centers or the central server 12. Aggregation of patient data is not needed. Communications between the central server 12 and the local processors 14, 18, 22 is of model values free of the medical data specific to any patient and in a human readable format.

The system of Figure 1 implements a rapid learning health care system. For example, rapid learning for care of patients is provided in a computer assisted theragnostics (CAT) system. This system may be used to supplement or even drive clinical trials in personalised medicine. This rapid learning health care system includes a set of institutions or organizations such as hospitals that are "linked" via a computer network such that the institutions can "share" predictive model data, such as parameters of a predictive model related to cancer patients, without sharing the actual patient data. The CAT system aims to create a set of coordinated, interoperable databases across multiple radiation oncology institutions in multiple countries and apply rapid learning across this network. A rapid learning community is feasible when it is supported by a system that addresses administrative, ethical and political barriers to sharing data. Such a community can be used to extract knowledge which is more accurate than the knowledge gained by individual centers. Rapid learning is implemented across multiple sites for effectively collecting data, aggregating data, implementing new insights, and evaluating outcomes, but while preserving patient privacy. Rapid learning in a distributed manner may overcome the data sharing barriers and allows learning from more diverse clinical data sets. Rapid learning allows for iterative adaptation of this knowledge as outcomes from new patients and new treatments become available. Rapid learning by using existing data in an automated or semi-automated manner may lead to the latest, validated insights being available for immediate implementation.

Figure 2 shows a method for learning a predictive model of medical knowledge. Distributed learning is used to preserve privacy. Patient data is handled by medical centers rather than collecting the patient data from different centers in one database. The medical center specific model statistics are communicated for reconciliation. The acts of the left and right columns represent acts by local medical centers. Two are shown in this example, but three or more may be used. The acts in the center column represent acts by a reconciliation device (e.g., central server). More than one reconciliation device may be used.

The method of Figure 2 is implemented by the system of Figure 1 or a different distributed learning system. Additional, different, or fewer acts may be provided. For example, act 40 is not provided, such as where the patient data is already available for training the predictive model.

In act 40, patient data is accessed. The patient data is clinical data, such as data gathered as routine in diagnosis and/or treatment of a patient. For example, the patient data includes billing records, physician notes, medical images, pharmacy database, lab records, and/or other information gathered about a patient. The patient data may include results, such as whether the patient still lives, whether there has been a reoccurrence, and/or whether further treatment or diagnosis occurred. The patient data that is routinely generated in patient care is re-used to extract and/or update medical evidence and knowledge. This has some possible benefits compared to controlled clinical trials due to the vast amount of patients for which data is available for machine learning. Patient data for patients who may usually be excluded from trials (e.g., due to advanced age, multiple co-morbidities, or concomitant medications) may be included in the learning.

The patient data is for a plurality of patients. The medical center collects patient data in a patient database. For each patient that visits, patient data is collected. For a given condition, there may be patient data for multiple (e.g., tens, hundreds, or thousands) patients.

Each medical center accesses patient data only for that medical center. Patient data for other medical centers is not accessed by a given medical center. This preserves the privacy of the patients even if the patient data is de-identified. De-identification is not relied on, limiting risk due to permitting access by others to patient data.

Since patient data for different medical centers is accessed by processors the respective different medical centers, the patient data being accessed is different. Due to the medical centers being in different geographic regions, different types of patients and/or different approaches to diagnosis and/or treatment are reflected in the patient data. For example, a medical center in Europe may draw from a different genetic, socio-economic, or type of patient group than a medical center in Africa. As another example, medical centers in different parts of a same city may draw from different types of patients. Differences in medical professionals may lead to differences in treatment or diagnosis at different medical centers.

The patient data is accessed by data mining. A data miner may be run using the Internet. A user may control the mining without access to patient data using a communications network. The data miner creates a database of structured clinical information relevant to the predictive model to be trained. The created structured clinical information may or may not also be accessed using the Internet.

The mining is performed using a domain knowledge base. The domain knowledge base may be encoded as an input to the system by manual programming or as machine-learnt programs that produce information that can be understood by the system. The data miner system uses the domain knowledge to determine what data to extract, how to extract the data, and how to determine the values for variables from the data.

The domain-specific criteria for mining the data sources may include institution-specific domain knowledge. For example, this may include information about the data available at a particular medical center, document structures at the medical center, policies of a medical center, guidelines of a medical center, and/or any variations of a medical center. The data miner is configured or programmed to access data at a given medical center. Data miners at different medical centers may be configured as appropriate for the respective medical center.

The domain-specific criteria may also include disease-specific domain knowledge. For example, the disease-specific domain knowledge may include various factors that influence risk of a disease, disease progression information, complications information, outcomes and variables related to a disease, measurements related to a disease, and policies and guidelines established by medical bodies.

In one embodiment, a data miner includes components for extracting information from the databases of patient data (computerized patient records), combining available evidence in a principled fashion over time, and drawing inferences from this combination process. The mined medical information may be stored in the structured CPR database. Any form of data mining may be used.

In one embodiment, the system will assimilate information from both imaging and non-imaging sources within the computerized patient record (CPR). These data can be automatically extracted, combined, and analyzed in a meaningful way, and the results presented. Such a system may also help avoid mistakes, as well as provide a novice with knowledge "captured" from expert users based on a domain knowledge base of a disease of interest and established clinical guidelines.

In one embodiment, the medical centers prevent access to the clinical data. Instead, a separate database that is a copy of the clinical database is used. The patient data in the copy may or may not be de-identified. For example, patient data is extracted in a de-identified manner to provide access for training a predictive model. The data extraction component hooks up to the site-specific patient data systems, extracts the desired data elements, de-identifies, and stores the resulting data elements in the local CAT system. Any one or more of open source tools (Talend Open Studio, Talend, Palo Alto, CA, USA and DIGITrans, MAASTRO Clinic, Maastricht, The Netherlands) may be used for extraction with de-identification. The extracted patient data is stored in a database, such as an SQL database or an open-source PACS (ClearCanvas, Toronto, ON, Canada). Other extraction or no extraction may be used.

The extraction is the same or different for each medical center. Since the medical centers may have different policies and/or computerized patient record systems, different extraction and/or access may be used.

In distributed learning, having the analysis, such as access and training in the form of software applications, come to the data, may result in different information representing the same concept. To provide for distributed learning, the patient data from the different medical centers is semantically normalized. This means that the environment in which the applications runs, the syntax of the data on which the applications work, and the meaning of the data elements are defined and controlled.

Each medical center may use unique and multiple information systems and differ in clinical practice including the way (e.g. language) in which data is collected. For semantic normalization, local (e.g., medical center) resources translate the local data to a semantic interoperable environment. The normalization is performed automatically to limit usage of medical center resources. Local medical center terms are semantically mapped to the CAT ontology. Any ontology may be used. For example, the CAT ontology includes the National Cancer Institute Thesaurus, which is accessed through the open source Jena framework. Additional concepts for radiotherapy authored in the open source Protégé editor (Stanford Center for Biomedical Informatics Research, Palo Alto, CA, USA) may be included. Additional or different ontologies or expansion of the current ontologies are easy to add if needed, such as for predictive modelling in non-cancer environments.

A specific term set to be used in the predictive modelling is selected or defined. Given terms in the local medical center, the ontology is used to associate the local terminology with the specific term set. Alternatively, the data is manually semantically normalized, such as by manual translation for extracting to the database to be accessed for training.

In act 42, a predictive model is trained. Machine learning is performed to train the predictive model based on the patient data. Any machine learning may be used. For example, a probabilistic boosting tree, support vector machine, or logistic regression model are trained. Using pre-defined or selected features, the patient data is used as training data. Since the outcome is known from the patient data for previously diagnosed or treated patients, the patient data represents a ground truth. Machine learning is performed on the patient data.

The machine learning creates statistical information correlating the features to outcome. The statistical information may be feature weights or counts learned from the patient data. The likelihood that a given feature indicates outcome is determined by a processor. For example, different features may be selected in an effort to determine inclusion and exclusion criteria for a possible clinical trail. As another example, the relative importance of different features as an indication of outcome for treatment is learned. The feature weighting may be used to predict two-year survival given a particular treatment. By in-silico trial simulation, a predictive model is learned for treatment, diagnosis, and/or clinical trial selection criteria.

The predictive model is learned separately at each local or different medical center. A processor derives model parameters by machine learning from the local patient data. Since different patient data is provided by the different medical centers, the parameters values or weights for the features may be different at the different medical centers. Due to differences in sample size (e.g., number of patients) at the different medical centers, the reliability of the learned model parameters may be different.

To aggregate the information, the medical center processors transmit the model parameters to a central or consensus server in act 44. Computer communications are used to transmit from processors at medical centers to a central server. The central server may be remote and may not be part of the medical center, so may not have access to patient data.

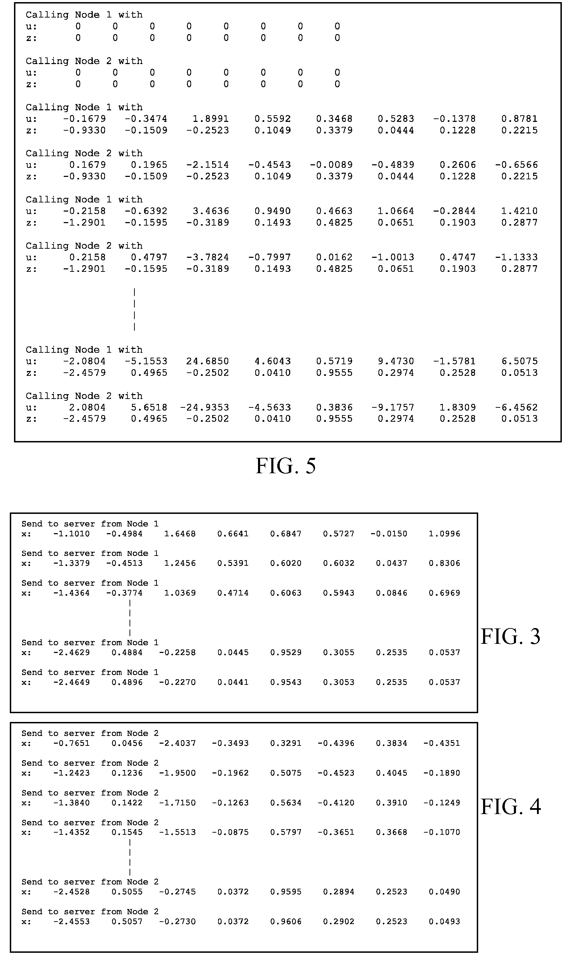

The parameters learned at each of the medical centers are transmitted. For example, Figures 3 and 4 show sample parameters as x values. A given line of the x values are parameters for a given predictive model. The values are different for the different nodes, where each node corresponds to a different medical center and corresponding patient data. The multiple rows of x values show iteration where each row represents a given message.

The learned statistical information is transmitted, not the patient data. For example, ten patients are treated with chemotherapy. The machine learning indicates that age is weighted as .37 indicator of two-year survival relative to six other features (e.g., gender, t-stage, n-stage, tumor location, Hb, and dose). Rather than transmitting the patients' ages, the .37 statistical value is transmitted. The statistical value derived from multiple patients is not restricted by privacy.

Keeping patient data inside the medical centers assures that local legal requirements, guidelines, procedures and infrastructure to ensure data privacy and security are satisfied. This requirement may lead to the approval of ethical and legal review bodies in multiple countries and legal systems. Also, medical centers still have full control of their patient data and what the patient data is used for, addressing the political barrier to share data.

To further assure compliance, the transmitted data may be in a message in a human readable format. The examples in Figures 3 and 4 are human readable. The message itself may be in an email, text, TCP/IP or other format, but may be rendered readable by a human using an application. An administrator may view the message and easily determine that no identifiable patient data is transmitted. The message is transmitted without any of the clinical information for any of the multiple patients. In alternative embodiments, the message is not human readable.

By learning locally and transmitting the learnt information, the analysis comes to the patient data, is transparent and is statistical in nature. If the data cannot come to the analysis, the rapid learning analysis comes to the patient data. For each medical center to control the use of the patient data, the incoming analysis is documented and reviewable before being accepted or rejected. Full control of the patient data is kept at the medical center. For ethical and/or privacy reasons, the output of the analyses is transparent to the institute and may only contain aggregate, statistical data.

In act 46, the central server receives the model parameters and does not receive any patient specific data. The parameters learned from different sets of patient data are received by one application. The values for the predictive model from the different processors are received. Since the same predictive model is being created at each medical center, the model values are for the same features. In the example of Figure 2, two different statistical values for "age" of the patient are received, one from each of the two different medical centers. Figures 3 and 4 show the values for eight features being in the received message.

Where the messages with the model parameters are in a human readable format, the server parses the messages and identifies the model parameters for specific features. The different model parameters from the different messages are identified.

In act 48, the central server reconciles the parameters from the different, local predictive models into a consensus model. Consensus model values are generated from the different sets of the model values. The server generates the consensus model values without access to the clinical data, instead relying on the statistical information. The feature weights learned from the different sets of patient data are used rather than the patient data.

Any distributed learning technique may be used. The server learns the predictive model by combining statistical information from other learnt predictive models. In one embodiment, an alternating direction of multipliers technique is used. The history of values from a same local prediction model is examined. The trend or change through multiple iterations of the statistic for a given feature is examined. A pre-determined, statistical, curve fit, or other direction to change the statistical value is determined, such as implementing a pattern of alternating directions (e.g., higher or lower) changes or selections of a next statistical value. The amount of change or step size is pre-determined, statistical, based on the curve fitting, or otherwise determined. Since trends from different local models are provided, the separately determined step sizes and/or directions are combined, such as by averaging. Any combination function may be used. Alternatively, curve fitting or other operation is performed on the statistical values from multiple of the local prediction models to determine a step size or reconciled value. The result is a statistical value for the given feature.

Where a history of previous learned values for the feature weights are not available, an initial value may be randomly set. A pre-determined value may be used. For example, Figure 5 shows null or "0" values for initial values of the model. These initial values are sent regardless of the statistical values received from the local models. Alternatively, the initial values are sent prior to learning by the local medical centers in act 42. In other embodiments, the local medical centers start with the initial values without communication from the central server. The central server combines the information from each local medical center, including the lack of fit values u, and aggregates to get the new consensus parameters z. Each local medical center maintains a "local" version of the model, with the input of the overall consensus model parameters z. Then, at each iteration, the local model is refined with respect to the local patient data at the local center, and the model fitting information is sent to the central server. Then, the central server combines this information from the local medical centers, reweights them based on the number of instances from each local center, and generates the new consensus parameters. The actual calculation depends on which algorithm (logistic regression, SVM, etc.) is adopted.

The consensus model is more generalized than any of the local predictive models. Since the consensus model has parameter values that are a function of information from multiple local predictive models, the consensus model is responsive to a broader range of medical data, resulting in more generalization. The local models, particularly in a first iteration but in later iterations as well, have parameters based entirely (first iteration) or primarily (subsequent iterations) on the medical data available locally, so are more specific. The consensus model incorporates information from a broader range of patients or diversity while the local models, other than steering by the consensus model, are trained by just the medical data available locally, which may represent a less diverse patient base.

In act 50, a check for completeness of the iterative process to generate the predictive model is performed. Any stop criterion may be used. For example, a particular number of iterations are performed. As another example, the change in the statistical values received from the local medical centers and the consensus value determined by the central server is sufficiently small (e.g., below a threshold difference) for each of the features and/or predictive models. Once the strop criterion is met, the consensus model is output as the predictive model to be used. This predictive model represents training from different patient data sets, but without transmission of any of the different sets from the local medical centers.

In act 52, the consensus model values are transmitted. For a next iteration, the reconciled model parameters are transmitted back to the local medical centers. The selected, averaged, alternating direction, or otherwise determined statistical values combined from the local predictive models are sent back. The message format is the same or different. The information transmitted does not include patient specific data since none is available to the central server.

Figure 5 represents an example message. The consensus model (e.g., model values) is represented by z. A lack of fit from the aggregate remote data is represented by u. The u values are an indication of the difference of the current model predictions on the local medical center patients and the actual outcomes of these patients. The u values are used to validate how good the current model fits with the local patient data, and are also sent to and used at the central server to generate the new consensus model.

In a repetition of acts 40 and 42, the consensus model values are used to re-train the local predictive models. The statistical values of the consensus model are used in the re-training. By accessing the patient data, the consensus predictive model is validated against the patient data. The validation indicates a level of match of the consensus predictive model with the patient data. The validation outputs modifications or re-learnt statistical values. The local medical centers each determine model values using the patient data, the consensus values, and the lack of fit values. Once the z value is sent to each local medical center, the local center retrains the model using only two pieces of information: the current consensus model z, and the local patient information. The current consensus model z acts like a "prior" to this local learning process. The local patient data is only accessible to this local process. Then, the newly learned local parameters are sent to the central server, without any patient specific information. Aggregation at the central server only needs these newly obtained local parameters, not any patient from any local server. Once again, different local or medical center specific model values are determined.

In the repetition of act 44, the new local model values are transmitted to the central server. The re-trained predictive models, in the form of the model parameters, are transmitted for reconciliation. In the repetition of act 48, another predictive model is created by reconciling the model values from the different local predictive models. The server learns from the statistics derived from the fit of the predictive model to different sets of patient data.

When complete, the resulting predictive model is formed based on statistics from the different sets of patient data. As a result, a larger training set size is provided for more reliable personalized prediction. This larger training set size is achieved without sharing the patient data outside the different medical centers storing the patient data.

Below is an example of community learning of a prediction model for survival in larynx cancer patients. A rapid learning community is formed based on the CAT system. This system meets three high level requirements: a) individual patient data never leaves the hospital, b) analysis comes to the data, is transparent and is statistical in nature, and c) semantic interoperability is achieved for the patients using limited resources. The CAT system is used to learn a prediction model for two-year survival of head and neck cancer patients treated with radiation therapy. Learning the model in individual institutes is compared to community learning, in which the model is learned in a distributed manner in data from two community members. The community learning is compared with a hypothetical learning setting where patient data from both community members are put together for learning (i.e., learning from aggregated patient data) in order to evaluate the accuracy of community learning.

The distributed learning component allows the execution of learning algorithms across institutes without the patient data ever leaving the individual institute. The component learns a model using the alternating direction method of multipliers and consists of one central, master application and distributed applications at the institutes that have agreed to participate in this specific learning request. In an iterative manner, the master application evaluates the learning results at each institute, provides updated model parameters for subsequent learning iterations, and decides when the optimal, consensus learning result has been achieved. The messages being exchanged between the central and the local CAT systems are human-readable and only contain aggregate information, like model parameters and counts. At the end of the learning procedure, each institute that participated has the consensus model available to them. The same distributed component may be used for model validation.

In this example use case in rapid learning, the CAT system learns a logistic regression model that predicts the probability of two year survival in larynx cancer patients treated with radiation therapy. The input parameters of the model are based on previously published work and are pre-determined as age, gender, pre-treatment haemoglobin, tumour stage, nodal stage, tumour location, and radiation dose.

The CAT system is installed at Maastricht Radiation Oncology, Maastricht, The Netherlands (MAASTRO) and at the Radiation Therapy Oncology Group, Philadelphia, PA, USA (RTOG). Then, two approaches for learning are compared, individual versus community learning. In individual learning, data from a single institute is used to train the model. In community learning, data from both institutes are used to train the model using the CAT distributed learning component. A total of three models are learned: From the MAASTRO dataset alone (M_{MAASTRO}), RTOG alone (M_{RTOG}) and from both institutes in a distributed manner: M_{COMMUNITY}. The models themselves, learning, and validation are implemented in Matlab (Mathworks, Natick, MA, USA), but other applications may be used.

The patient data used in this study originates from previously published work on larynx cancer from both institutes. The MAASTRO dataset consisted of 969 larynx cancer patients treated with radiotherapy alone until 2008. This is a routine, clinical population consisting of all laryngeal cancer patients treated with curative intent in that time frame for which electronic data was available. At RTOG, data on 194 larynx cancer patients is available. This is a heavily selected, controlled clinical trial patient population. Table 1 gives an overview of the patient characteristics.

**Table 1: Patient characteristics**

| | | MAASTRO (N=969) | % | RTOG (N=194) | % |
|---|---|---|---|---|---|
| | | | | | |
| Age | <=60 | 355 | 37% | 129 | 66% |
| | >60 | 614 | 63% | 65 | 34% |
| | | | | | |
| Gender | Male | 861 | 89% | 155 | 80% |
| | Female | 108 | 11% | 39 | 20% |
| | | | | | |
| T-Stage | T1 | 519 | 54% | 2 | 1% |
| | T2 | 258 | 27% | 30 | 15% |
| | T3 | 126 | 13% | 122 | 63% |
| | T4 | 66 | 7% | 40 | 21% |
| | | | | | |
| N-Stage | N0 | 875 | 90% | 59 | 30% |
| | N+ | 94 | 10% | 135 | 70% |
| | | | | | |
| Tumour Location | Glottic | 716 | 74% | 45 | 23% |
| | Non-glottic | 253 | 26% | 149 | 77% |
| | | | | | |
| Hb [mmol/L] | <8.5 | 184 | 19% | 88 | 45% |
| | >=8.5 | 785 | 81% | 106 | 55% |
| | | | | | |
| Dose [Gray] | <=66 | 597 | 62% | 6 | 3% |
| | >66 | 372 | 38% | 188 | 97% |
| | | | | | |
| Treatment | Radiation alone | 969 | 100% | 0 | 0% |
| | Chemoradiation | 0 | 0% | 194 | 100% |
| | | | | | |
| Two year survival | Yes | 829 | 86% | 131 | 68% |
| | No | 140 | 14% | 63 | 32% |

| | | | | | |
|---|---|---|---|---|---|
| Dose: Prescribed physical dose; Hb: Haemoglobin | | | | | |

The two institutions and a central server form a rapid learning community that learns and shares knowledge. By comparing the predictive models, it may be shown that rapid learning allows knowledge to be extracted from coordinated databases of routine care and clinical trial data sources. Rapid learning may be done without data leaving the institute that holds the data and without the institute losing control of the data, addressing the need for secured and trusted use of these data. This approach balances the general willingness and realization that a community provides for better rapid learning with the legitimate concerns of individual institutes to share data from an administrative, ethical and political perspective.

The design of the underlying technology, the CAT system, combines a local semantic interoperable environment with a distributed learning framework. This combination makes community learning possible across institutes and countries. At the end of the learning process, the consensus knowledge is per design available to the community, which can then validate the knowledge locally and can apply this knowledge immediately or not. When new patients (or new members) in the community become available, an updated model may be learned. The process is repeated and/or further iterations performed, but with the additional training data.

This community approach is different from efforts that focus on individual health systems. Furthermore, no single institute or country may have enough patients coupled with enough diversity in patients and treatments to learn how different treatments affect outcome in an individual patient. Other initiatives require data to move from the data holder to the data user. The largest initiative is the USA-based caBIG program, which is designed as a federated system for research but has not reached the level of semantic interoperability to perform learning on clinical data with no patient data sharing. On the European side, Health-e-Child provides an integrated biomedical platform for paediatric applications that was able to integrate heterogeneous data from multiple countries, but again data, in this case of only a limited number of subjects, was de-identified and sent around between institutes. The requirement for institutes to release their data limits the number of patients and data elements institutes are willing and able to share.

Rapid learning is a new field in which the ethical aspects and especially the need for informed consent have not been fully addressed and differ between countries. What can be said is that this community-based rapid learning type of research meets all the conditions of the so-called American Common Rule for waivability of informed consent: (a) the research involves no more than minimal risk; (b) the waiver will not adversely affect the rights and welfare of the subjects; (c) the research could not practicably be carried out without the waiver; and (d) whenever appropriate, the subjects will be provided with additional pertinent information after participation. The patients may or may not be required to consent to use of their data. Of course any waiver of consent does not discharge institutes from any obligation to properly inform patients on the use of a rapid learning system and to remove patients who object to their use of data from such a system. It should also be stressed that anytime an intervention or change in practice is planned, this should be clearly identified as such and has to be split (in a regulatory sense) from the rapid learning system.

In this example, the prediction model is trained for a very specific question: "What radiation dose should this larynx cancer patient receive for an expected survival ofX% at two years." This example question is of the type of questions that are being posed at the point of care on a regular basis. A simple, transparent model (logistic regression) is used. The six input parameters are selected to focus on the community aspect of learning and validation of the model, rather than the model itself. As a consequence, the model performance in terms of discrimination is poor (Table 3). It is expected that learning models through more advanced machine learning algorithms, such as support vector machines or Bayesian networks, adding additional input parameters (e.g. from imaging and biology) and performing feature selection as part of learning may lead to better performing models.

This example application shows that patient populations across the community can be very different (Table 1). In this application, a routine, unselected routine care population from the Netherlands is mixed with a controlled clinical trial population from the USA. Although extreme in this case, in rapid learning, one cannot expect patient populations to match well, and this has important consequences. On a positive note, it shows one can learn something from such very different datasets (AUC of both M_{MAASTRO} and M_{RTOG} is higher than 0.5 when validated at RTOG and MAASTRO, respectively). Furthermore, a community model is more generalizable, as seen by the higher value of the AUC of M_{COMMUNITY} vs. the individual models. The community model should be carefully validated at the institute level to make sure that the derived knowledge can be applied locally (Table 3 and Figure 1). In the distributed approach, two models are available to the participating center: the model learned on the institutes own data (the first iteration) and the community model learned after many iterations. After the learning process, models can be validated with the institute's own data, hopefully providing the insight and the confidence in the models for them to be applied at the point of care to change a decision. For the latter, a further performance assessment in terms of calibration and decision-analytic measures may be performed.

Community learning led to a prediction model that performed significantly better than a model based on learning the model from data from individual institutes (community learning yields test Area Under the Curve (AUC) of 0.662, and models learned using individual institute data yield test AUC of 0.609 and 0.652, respectively). Compared to the hypothetical setting of putting all patient data together for learning, the community learning algorithm yields an AUC difference less than 10⁻¹⁵, which indicates that the two models are almost identical.

Additionally, more site-adaptive designs may improve these algorithms. For instance, if there are certain data or variables missing from a certain site, site specific missing data imputation methods may leverage the data characteristic from the site. For example, an average, null, median, expected or other substitute value is used for any missing data. If there is a known distribution shift (i.e. for the same variables, their value distributions are not the same across multiple sites), transfer learning or domain adaptation methods may account for the shift. This would lead to knowledge sharing across multiple sites and at the same time site specific parameter fitting for each individual site.

The rapid learning system (i) captures data systematically; (ii) analyzes collected data retrospectively and/or prospectively; (iii) implements findings into subsequent clinical care; (iv) evaluates resulting clinical outcomes; and/or (v) generates additional hypotheses for future investigation. The prediction models may be extended and/or updated to include more patients, treatment modalities (e.g. surgery, chemotherapy, targeted therapy), input parameters (e.g., smoker or not), different outcomes (e.g. patient-reported quality of life outcomes), and/or prospectively validate the models in terms of performance and the impact on treatment decisions.

The CAT system was reviewed by seven institutes from five countries (Netherlands, Belgium, Italy, Germany and USA). In all cases the CAT system was considered to be completely in accordance with regulations. At each institute in which data from patients was to be included without a per-patient informed consent, the internal review boards (IRB) were asked for their opinion on this matter. In all cases the, IRB responded that this was allowed. In one instance, an insurance to protect against a privacy breach was requested.

To access the patient data for learning, patient data from one or more sources is provided. Since some sources may be unstructured, providing for mining from the unstructured data or from both structured and unstructured data may allow access to more reliable, more comprehensive, and/or more consistent information. By understanding natural language, the unstructured data may be analyzed and understood in order to convert salient pieces of information into structured fields for training. The system mines through the patient record and identifies inconsistent information. Such identification and data mining may be by the REMIND™ system. Such system is shown and described in US patent numbers 7,617,078, 7,181,375, 7,744,540, 7,457,731 and US 7,840,511, as well as US application serial numbers 10/287,075, 10/287,098, 10/287,054, 10/287,329, 10/287,074, 10/287,073, 11/435,660, 11/435,657, 11/758,716, 12/488,083, 12/780,012, 10/319,365, 12/190,675. Other data mining may be used.

In one example embodiment, a plurality of electronic medical records for a particular patient or set of patients are provided at each medical center. These records contain both structured and unstructured data. For example, the medical records for a given patient may contain a physician's "free text" notes taken during the patient's visits. The records may also comprise structured information such as "Q and A" documentation provided by the patient, a nurse, doctor, or other. Such information may include a questionnaire having "yes" or "no" questions as well as space for explanations.

This medical information may be accessed by a data miner having a domain knowledge base. The data miner may include an extraction component for extracting information from the data sources to create a set of probabilistic assertions, a combination component for combining the set of probabilistic assertions to create one or more unified probabilistic assertion, and an inference component for inferring patient states from the one or more unified probabilistic assertion.

Unified probabilistic assertions are mined from information relevant to the predictive model being formed based on domain-specific criteria. The domain-specific criteria may be specific to cancer, lung cancer, symptoms, whether the patient is a smoker, or other considerations. As described in the aforementioned REMIND^{™} patents and applications, the system is able to search, mine, extrapolate, combine, and/or process data that is in an unstructured format. In one example, the domain knowledge base, contains a list of disease-associated terms or other medical concepts or terms, and can mine for corresponding information from a medical record. The domain knowledge base may automatically mine this information where the mining is based on probabilistic modeling and reasoning. For example, for a medical concept such as "heart failure," the processor automatically determines the odds that heart failure has indeed occurred or not occurred in the given patient based on a transcribed text passage. In this example, the concept is "heart failure" and the states are "occurred" and "not occurred." In the system, these tasks may be carried out by a processor.

The mining may be used to determine values of input features for modeling. Alternatively or additionally, the mining may be used to determine a ground truth (e.g., outcome) for machine training based on diagnosed and/or treated patients.

In one embodiment, a probabilistic methodology is used to infer the state of the patient. This is described in US 7,840,511, which is incorporated by reference in its entirety. A probabilistic model takes into account the statistics of words or words and their relationship to patient states and conditions. There are many variables, some known and others unknown, that can influence the meaning of a sentence, and their relationship and combined effect is clearly not deterministic. Medical concepts cannot be easily inferred from words or phrases alone, such as in phrase spotting, since the language employed is usually complex and unstructured from a computational perspective.

Once the unstructured information is extrapolated from the medical records, it may or may not be put into a structured format such as a database or spreadsheet. Regardless, the system and/or method then assign "values" to the information. These values may be labels as described in US 7,840,511. In one embodiment, text passages from the medical data are grouped into concepts. Example medical concepts could be 'Congestive Heart Failure', 'Cardiomyopathy', or 'Any Intervention.' The outcome of this analysis will be at the sentence, paragraph, document, or patient file level. For example, the probability that a document indicates that the medical concept or concepts of interest are satisfied ('True') or not ('False') is modeled. The model may be based on one level (e.g., sentence) for determining the state at a higher or more comprehensive level (e.g., paragraph, document, or patient record). The state space is Boolean (e.g., true or false) or any discrete set of three or more options (e.g., large, medium and small). Boolean states spaces may be augmented with the neutral state (here referred to as the 'Unknown' state).

In another embodiment, a probabilistic model, such as that described in US 7,840,511, assigns labels to data in the medical records. The values for variables representing the state of the patient may be determined.

The labels for the concepts can then be compared to determine if there is any inconsistent or duplicate information. For example, if a patient has indicated in a questionnaire that he or she is not a smoker, in one part, the system will generate a label showing that smoker = no. However, if a doctor has noted in his or her notes that the person is a smoker, in another part of the records it will show a label that smoker = yes. This situation may arise where the patient has recently quit smoking or where there is an inaccuracy. These labels would conflict. The system would identify and report this anomaly. The system would also identify and report if there were two instances where it was indicated "smoker = no". This would be identified as duplicate information. The inconsistency may be resolved by temporal analysis and/or by probabilistic analysis (e.g., 75% chance the patient is a smoker based on knowledge about patient accuracy in reporting smoking and physician accuracy in noting smoking).

As another example, consider the situation where a statement such as "The patient has metastatic cancer" is found in a doctor's note, and it is concluded from that statement that <cancer=True (probability=0.9)>. (Note that this is equivalent to asserting that <cancer=True (probability=0.9), cancer=unknown (probability=0.1)>).

Now, further assume that there is a base probability of cancer <cancer=True (probability=0.35), cancer=False (probability=0.65)>(e- .g., 35% of patients have cancer). Then, this assertion is combined with the base probability of cancer to obtain, for example, the assertion <cancer=True (probability=0.93), cancer=False (probability=0.07)>.

However, there may be conflicting evidence. For example, another record or the same record may state that the patient does not have cancer. Here, we may have, for example, <cancer=False (probability=0.7). The system and method of the present invention would be able to identify this instance, report it to a user, and determine a most probable value.

In mining the patient data for training the predictive model, the processor may receive medical transcript information. The medical transcript is a text passage, such as unstructured, natural language information from a medical professional. Unstructured information may include ASCII text strings, image information in DICOM (Digital Imaging and Communication in Medicine) format, or text documents. The text passage is a sentence, group of sentences, paragraph, group ofparagraphs, document, group of documents, or combinations thereof. The text passage is for a patient. Text passages for multiple patients may be used.

The state of the patient related to one or more medical concepts is determined from the text passage. Multiple states for a respective multiple medical concepts may be determined for a given text passage. Alternatively, the most probable medical concept and corresponding state are identified.

The user input, network interface, or external storage may operate as an input operable to receive user identification of the medical transcript. For example, the user enters the text passage by typing on a keyboard. As another example, a stored file in a database is selected in response to user input. In alternative embodiments, the processor automatically processes text passages, such as identifying any newly entered text passages and processing them.

For application of the probabilistic model used in mining, the processor may receive the text passage from a medical transcript. The probabilistic model is applied to the text passage of the medical transcript. Key terms are identified in the text passage, such as identifying a discrete set of terms as elements identified as a function of mutual information criteria. The key terms are associated with learned statistics of words or phrases relative to the state of the medical concept of interest. Based on the statistics for conditional and prior probability functions of words or phrases relative to the state or a discrimitively-learnt model, a state with a highest probability given the terms identified in the text passage is determined. In one embodiment, negation and/or modifier terms are identified and input to the model separately from the key terms of a medical concept. A Bayes or other model has a summary node for the text passage, a negation node, and a modifier node. The state is inferred as a function of an output from the probabilistic model applied to the text passage.

Based on the application of the probabilistic model, the processor outputs a state. The state may be a most likely state. A plurality of states associated with different medical concepts may be output. A probability associated with the most likely state may be output. A probability distribution of likelihoods of the different possible states may be output.

The processor outputs the state and/or associated information on the display, into a memory, over a network, to a printer, or in another media. The display is text, graphical, or other display. The display is operable to output to a user a state associated with a patient. The state provides an indication of whether a medical concept is indicated in the medical transcript. The state may be whether a disease, condition, symptom, or test result is indicated. In one embodiment, the state is limited to true and false, or true, false and unknown. In other embodiments, the state may be a level of a range of levels or other non-Boolean state.

It is to be understood that the present embodiments may be implemented in various forms of hardware, software, firmware, special purpose processors, or a combination thereof. Preferably, the present embodiments are implemented in software as a program tangibly embodied on a program storage device. The program may be uploaded to, and executed by, a machine comprising any suitable architecture. Preferably, the machine is implemented on a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the program (or combination thereof) which is executed via the operating system. In addition, various other peripheral devices may be connected to the computer platform such as an additional data storage device and a printing device.

It is to be understood that, because some of the constituent system components and method steps are preferably implemented in software, the actual connections between the system components (or the process steps) may differ depending upon the manner in which the present invention is programmed.

While this invention has been described in conjunction with the specific embodiments outlined above, it is evident that many alternatives, modifications, and variations will be apparent to those skilled in the art. Accordingly, the preferred embodiments of the invention as set forth above are intended to be illustrative, not limiting. A variety of modifications to the embodiments described will be apparent to those skilled in the art from the disclosure provided herein. Thus, the present invention may be embodied in other specific forms without departing from the spirit or essential attributes thereof.

## Claims

1. A method for learning predictive models of medical knowledge, the method comprising:
accessing (40) first patient data in a first database of a first medical center;
training (42), by a first processor of the first medical center, a first predictive model with the first patient data;
transmitting (44) first parameters of the first predictive model without transmitting the first patient data, the transmitting being to a server remote from the first medical center and a second medical centers;
accessing (40) second patient data in a second database of the second medical center different than the first medical center;
training (42), by a second processor of the second medical center, a second predictive model with the second patient data;
transmitting (44) second parameters of the second predictive model without transmitting the second patient data, the transmitting being to the server;
reconciling (48), by the server, the first and second parameters into a third predictive model;
transmitting (52) third parameters of the third predictive model to the first and second medical centers;
re-training (42) the first and second predictive models at the first and second medical centers, respectively, as a function of the third parameters;
transmitting (44) fourth and fifth parameters of the re-trained first and second predictive models to the server; and
generating (50), by the server, a fourth predictive model as a function of the fourth and fifth parameters.

2. The method of claim 1 wherein accessing (40) the first and second patient data comprises accessing (40) data of multiple patients of the first medical center and data of multiple patients of the second medical center, the multiple patients being different patients that have been treated for a same condition, and the first medical center being in a different geographic region than the second medical center.

3. The method of claim 1 wherein accessing (40) comprises semantically normalizing the first and second patient data at the first and second medical centers to a common ontology.

4. The method of claim 1 wherein re-training (42) the first and second predictive models, reconciling (48) into the third predictive model, and generating the fourth predictive model each comprise machine learning a logistic regression model where the third, fourth and fifth parameters comprise feature weights learned from the first and second patient data.

5. The method of claim 1 wherein generating (50) the fourth predictive model comprises generating the fourth predictive model as a function of both first and second patient data without the first and second patient data having left the first and second medical centers, respectively.

6. The method of claim 1 wherein training (42), re-training (42) the first and second predictive models, reconciling (48) into the third predictive model, and generating (50) the fourth predictive model comprise simulating an in-silico trial for a treatment.

7. The method of claim 1 wherein training (42), re-training (42) the first and second predictive models, reconciling (48) into the third predictive model, and generating (50) the fourth predictive model comprise simulating an in-silico trial for a clinical trail selection criteria.

8. The method of claim 1 wherein training (42), re-training (42) the first and second predictive models, reconciling (48) into the third predictive model, and generating (50) the fourth predictive model comprise modeling probability of survival.

9. The method of claim 1 wherein reconciling (48) comprises performing alternating direction of multipliers.

10. The method of claim 1 wherein transmitting (44) the first, second, fourth, and fifth parameters comprises transmitting statistical information derived from the first and second patient data.

11. The method of claim 1 wherein the first and second patient data includes clinical information for multiple patients, and wherein transmitting (44) the first, second, fourth, and fifth parameters comprises transmitting a message without any of the clinical information for any of the multiple patients.

12. The method of claim 1 wherein transmitting (44) the first, second, third, fourth, and fifth parameters comprises transmitting in a human readable format.

13. The method of claim 1 wherein training (42), reconciling (48), re-training (42) and generating comprise distributed learning, wherein re-training (42) comprises validating the third parameters against the first and second patient data at the first and second medical centers, respectively, and wherein generating comprises determining satisfaction of a stop criterion by a consensus between the first and second predictive models from the fourth and fifth parameters.

14. In a non-transitory computer readable storage medium having stored therein data representing instructions executable by a programmed processor for learning a predictive model of medical knowledge, the storage medium comprising instructions for:
receiving (46) different sets of model values for the predictive model from different processors, the different sets of the model values from the different processors being machine learnt from clinical data for different sets of patients, the clinical data for the different sets of the patients not being received;
generating (48) consensus model values from the different sets of the model values without access to the clinical data; and
transmitting (52) the consensus model values to the different processors.

15. The non-transitory computer readable storage medium of claim 14 wherein receiving (46) comprises receiving (46) the model values for multipliers of the predictive model, the model values representing statistics derived from the clinical data of the respective set of patients, wherein generating (48) the consensus model values comprises alternating direction of the multipliers.

16. The non-transitory computer readable storage medium of claim 14 wherein receiving (46), generating (48), and transmitting (52) are performed iteratively until a stop criteria is satisfied.

17. The non-transitory computer readable storage medium of claim 14 wherein receiving (46) comprises receiving (46) the different sets of the model values in a human readable format.

18. A system for learning a predictive model of medical knowledge, the system comprising:
a central server (12); and
a plurality of processors (14) for a respective plurality of different medical entities, each of the processors configured to generate local predictive models from medical data of the respective medical entity;
wherein the central server (12) and processors (14) are configured to perform distributed machine learning using the medical data from the different medical entities, the distributed machine learning resulting in a central predictive model learnt from the medical data of the plurality of the different medical entities while avoiding transfer of the medical data from any of the different medical entities.

19. The system of claim 18 wherein the processors (14) are configured to generate model statistics representing the local predictive models, wherein the processors (14) are configured to communicate the model statistics and not communicate the medical data to the central server (12), and wherein the central server (12) is configured to generate the central predictive model from the model statistics.

20. The system of claim 18 wherein the processors (14) are configured to semantically normalize the medical data at the respective medical entities prior to performing the distributed machine learning, wherein communications between the central server (12) and the local processors (14) comprises model values free of the medical data specific to any patient and in a human readable format.

21. The system of claim 18 wherein the central predictive model is more generalized than any of the local predictive models.

22. A method for learning a predictive model of medical knowledge, the method comprising:
accessing (40) first patient data in a first database of a first medical center;
analyzing (42), by a first processor of the first medical center, the first patient data;
transmitting (44) first aggregate statistical data resulting from the analyzing without transmitting the first patient data, the transmitting being to a server remote from the first medical center and a second medical centers;
accessing (40) second patient data in a second database of the second medical center different than the first medical center;
analyzing (42), by a second processor of the second medical center, the second patient data;
transmitting (44) second aggregate statistical data resulting from the analyzing without transmitting the second patient data, the transmitting being to the server; and
reconciling (48), by the server, the first and second aggregate statistical data into a predictive model.
